# EUROPEAN PATENT APPLICATION

(11) **EP 4 252 743 A1**
(43) Date of publication of application: **04.10.2023**
(21) Application number: 21898239.5
(22) Date of filing: 29.04.2021
(51) Int. Cl.: A61K 9/127, A61K 9/50, A61K 38/16, A61K 45/06, A61P 35/00

(54) **EXOSOMES EXPRESSING RSVF AND USE THEREOF**

(30) Priority: 27.11.2020 KR 20200163200
(71) Applicant: Korea Institute of Science and Technology, Seoul 02792 (KR)
(72) Inventor: KIM, In-San, Seoul 02792 (KR); KIM, Gi-beom, Seoul 02792 (KR); NAM, Gi-hoon, Seoul 02792 (KR); KIM, Seonghyun, Gimpo-si, Gyeonggi-do 10129 (KR); YANG, Yoosoo, Seoul 02792 (KR)
(74) Representative: Longland, Emma Louise
(86) International application number: PCT/KR2021/005419
(87) International publication number: WO 2022/114409

(57) **Abstract**

Provided are an exosome expressing a respiratory syncytial virus F protein (RSVF) and use thereof.

The exosome expressing the respiratory syncytial virus F protein (RSVF) according to the present invention kills cancer cells by specifically fusing to membranes of cancer cells, and suppresses tumor growth by accumulating in a tumor specific manner, and accordingly, it may be used in the prevention or treatment of cancer.

## Description

### [Technical Field]

The present invention relates to an exosome expressing a respiratory syncytial virus F protein (RSVF) and use thereof.

### [Background Art]

Oncolytic virus (OV) therapy began in the late 19^{th} century when wild-type influenza virus was used to treat leukemia. OV therapy has made remarkable advances and achievements in the treatment of tumors. In particular, *Talimogene laherparepvec* (T-VEC) received approval from the US Food and Drug Administration (FDA) in 2015 for the treatment of melanoma. This has shown promise for OV therapy, and about 60 clinical trials using various oncolytic viruses are being conducted. The biggest feature of OVs is that they have no effect on healthy cells and adversely affect malignant cells.

As described, OVs have exhibited antitumor effects against various cancers, but depending on their origin, they are neutralized by antibodies and the innate immune system and rapidly eliminated from the blood. For this reason, there are problems in that OVs are difficult to apply to systemic treatment, and they can be applied only to limited tumors where intratumoral injection is easy, such as melanoma and head and neck cancer (M.S. Ferguson, et al., Systemic Delivery of Oncolytic Viruses: Hopes and Hurdles, Advances in Virology, vol. 2012, Article ID 805629, 14 pages, 2012.).

Respiratory syncytial virus (RSV) belonging to the genus *Orthopneumovirus* is a negative single-stranded RNA virus that infects the lower respiratory tract and causes pneumonia and bronchitis. RSV consists of 11 proteins, among which an RSV fusion protein (RSVF) is known to play an essential role in infecting host cells (S. Bueno, et al., Host immunity during RSV pathogenesis, International Immunopharmacology. Volume 8, Issue 10, October 2008, Pages 1320-1329; J.S. McLellan, et al., Structure and Function of Respiratory Syncytial Virus Surface Glycoproteins, In: Anderson L., Graham B. (eds) Challenges and Opportunities for Respiratory Syncytial Virus Vaccines. Current Topics in Microbiology and Immunology, vol 372. Springer, Berlin, Heidelberg.). Various attempts have been made to use RSV as an oncolytic virus, based on the fact that RSVF fuses with target cells to cause cell death and has shown significant tumor regression effects in preclinical studies. In addition, nucleolin, which is a major receptor for RSVF, is known to be highly expressed on the surface of tumor cells, and thus it has been widely used as a receptor for tumor-targeting drugs.

Despite these attempts, OV therapy using RSV still could not overcome the limitations of OV therapy in that OV is rapidly eliminated from the blood.

On the other hand, exosomes, which have attracted considerable attention as effective drug delivery vehicles, are nano-sized extracellular vesicles released by most cell types, and contain various biomolecules of the origin, such as RNA, proteins, and lipids. Considering that exosomes participate in cell-to-cell signaling and have a long circulation time in the blood without toxicity, they may be used as effective cell-derived carriers and may exhibit remarkable effects in cancer treatment. In addition, it is known that exosomes are suitable for delivering biologically active membrane proteins because they have a phospholipid bilayer.

### [Disclosure]

### [Technical Problem]

The present inventors have prepared an oncolytic exosome expressing RSVF on the surface thereof, and they found that the prepared RSVF-expressing exosome (RSVF-Exo) may be preferentially fused with malignant cells in a nucleolin-dependent manner, the nucleolin which is a major receptor for RSVF and is expressed on the surface of tumor cells in a high level, and may additionally cause immunogenic cell death (ICD) through an endoplasmic reticulum (ER) stress-mediated pathway. In addition, when RSVF-Exo is fused with tumors, viral proteins are expressed on the surface thereof and readily recognized by the immune system, resulting in increased phagocytosis. It was also found that systemic injection of RSVF-Exo accumulated at the tumor site showed anti-tumor effects by inducing upregulation of the immune system, and when applied together with an immune checkpoint blocking therapy, RSVF-Exo synergistically improved the efficacy of immune checkpoint blockade, thereby completing the present invention.

### [Technical Solution]

An object of the present invention is to provide an exosome expressing a respiratory syncytial virus F protein (RSVF).

Another object of the present invention is to provide a pharmaceutical composition for preventing or treating cancer, the pharmaceutical composition including the exosome as an active ingredient.

Still another object of the present invention is to provide a vaccine composition for preventing cancer, the vaccine composition including the exosome as an active ingredient.

Still another object of the present invention is to provide a method of treating cancer, the method including the step of administering the pharmaceutical composition to an individual.

Still another object of the present invention is to provide a kit for preventing or treating cancer, the kit including a first composition including the exosome expressing the respiratory syncytial virus F protein; and a second composition including an anticancer agent as an active ingredient.

Still another object of the present invention is to provide a method of preparing the exosome expressing the respiratory syncytial virus F protein, the method including the steps of i) transfecting cells with a plasmid including a polynucleotide encoding the respiratory syncytial virus F protein (RSVF); ii) treating the cells of the step i) with a drug; and iii) separating the exosome from the supernatant of the cells of the step ii).

Still another object of the present invention is to provide use of the exosome in the prevention or treatment of cancer.

### [Advantageous Effects]

An exosome expressing a respiratory syncytial virus F protein (RSVF) according to the present invention kills cancer cells by specifically fusing to membranes of cancer cells, and suppresses tumor growth by accumulating in a tumor specific manner, and accordingly, it may be used in the prevention or treatment of cancer.

### [Brief Description of Drawings]

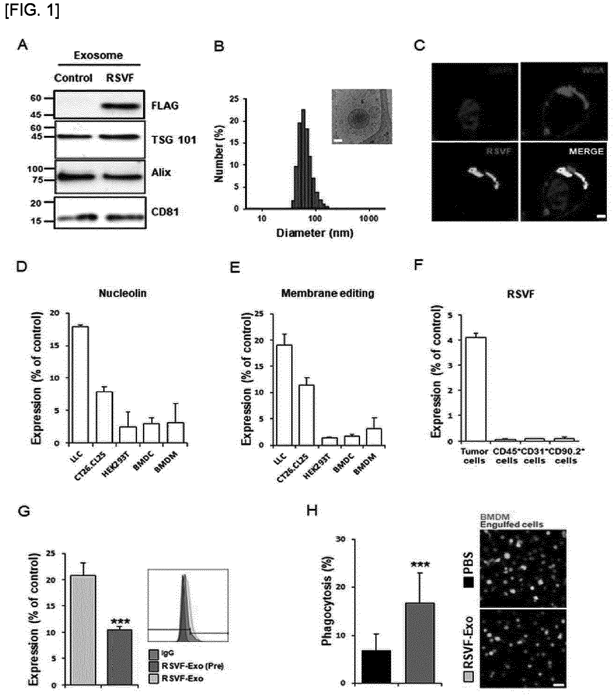

FIG. 1 shows characteristics and fusion activity of nucleolin-dependent respiratory syncytial virus F protein (RSVF)-expressing exosome (RSVF-Exo). (A) RSVF expression and detection results of exosome markers TSG101, Alix, and CD81 in RSVF-Exo. (B) Size distribution and cryo-TEM image of RSVF-Exo. (C) Images showing fusion of RSVF-Exo to cancer cells. (D) Graph showing expression levels of nucleolin in LLC, CT26.CL25, HEK293T, BMDC, and BMDM. (n = 4). (E) After treatment of LLC, CT26.CL25, HEK293T, BMDC, and BMDM with exosomes, the amount (n = 4) of RSVF on the surface of each cell. (F) Relative mean fluorescence intensity (MFI) of RSVF on the surface of CD45+, CD31+, and CD90.2+ cells in tumors which were separated 2 hours after intratumoral injection of RSVF-Exo, Con-Exo, and PBS on days 10 and 11 after inoculation of tumor cells into CT26.CL25-mCherry tumor-bearing mice (n = 3; *** p <0.001; ANOVA with Turkey's post-hoc test.). (G) RSVF expression levels on the surface of CT26.CL25 cells, after pre-blocking of the cells with a nucleolin antibody and treatment with RSVF-Exo (n = 3; *** p <0.001; ANOVA with Student's t-test.). (H) Diagram confirming that RSVF exposed on the surface of cancer cells acts as a PAMP signal to increase phagocytosis of macrophages.

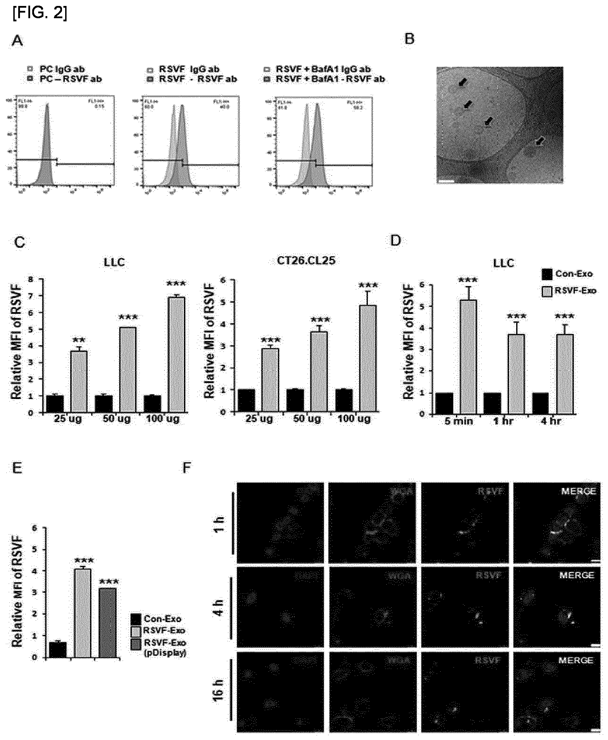

FIG. 2 shows characteristics and fusion activity of RSVF-Exo. (A) RSVF expression levels in RSVF-Exo. (B) RSVF-Exo identified by cryo-electron microscopy (Cryo-TEM). (C) Relative MFI of RSVF on LLC and CT26.CL25 cell membranes at each exosome treatment concentration. (D) Relative MFI of RSVF on the LLC cell membrane at each exosome treatment time. (E) Degree of fusion of exosomes (RSVF-Exo(pDisplay) expressing RSVF without a cytoplasmic domain included in a pDisplay vector and exosomes (RSVF-Exo) expressing the entirety of RSVF to LLC cells. (F) Degree of fusion of RSVF-Exo on the tumor cell surface over time, as examined by confocal microscopy.

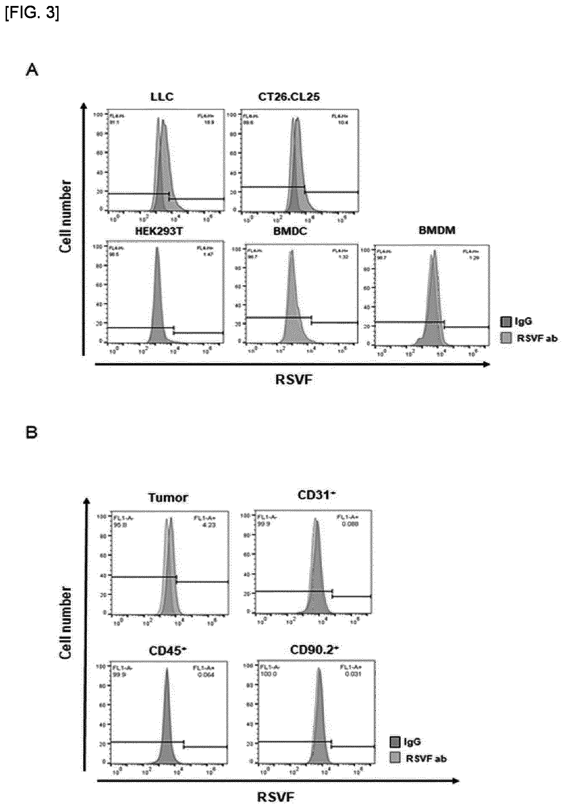

FIG. 3 shows analysis of the amount of RSVF on the surface of each cell fused with RSVF-Exo *in vitro* (A) or *in vivo* (B).

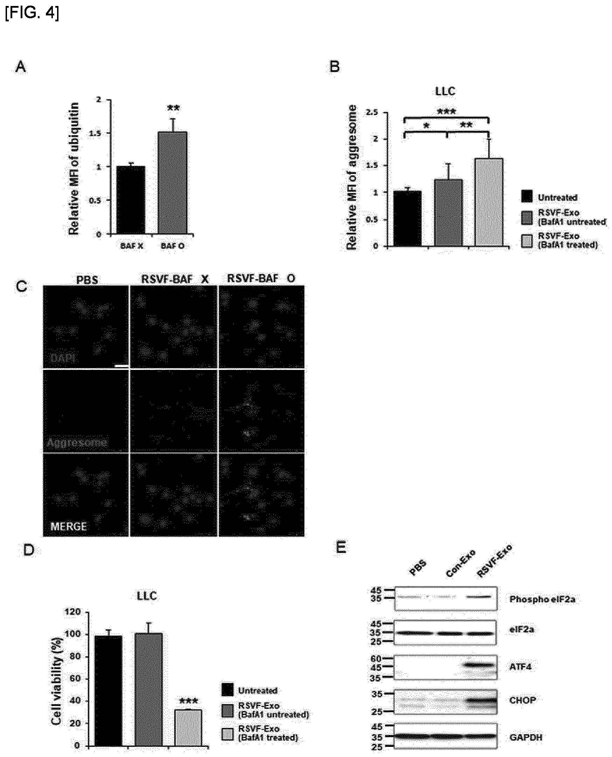

FIG. 4 shows immunogenic cell death of tumor cells through endoplasmic reticulum (ER) stress of RSVF-Exo. (A) The amount of ubiquitinated proteins of RSVF-Exo extracted with or without bafilomycin A1 treatment. (B) FACS analysis of aggresomes, which are unfolded proteins, inside LLC cells, after fusion of LLC cells with RSVF-Exo extracted with or without bafilomycin A1 treatment. (C) Results of confocal microscopy analysis of examining the presence of aggresomes inside CT26.CL25 cells. (D) Degree of death of LLC cells by RSVF-Exo extracted with or without bafilomycin A1 treatment. (E) Results of western blotting of intracellular ER stress markers after treating LLC cells with a control exosome (Con-Exo) or RSVF-Exo extracted after bafilomycin A1 treatment.

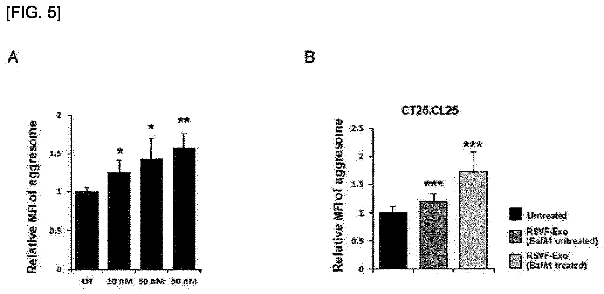

FIG. 5A shows the amount of ubiquitinated proteins of RSVF-Exo inside HEK293T cells, which are parental cells, according to each treatment concentration of bafilomycin A1 (10 nM, 30 nM, 50 nM), and FIG. 5B shows FACS analysis of aggresomes of unfolded proteins inside CT26.CL25 cells after fusion of CT26.CL25 cells with RSVF-Exo extracted with or without bafilomycin A1 treatment.

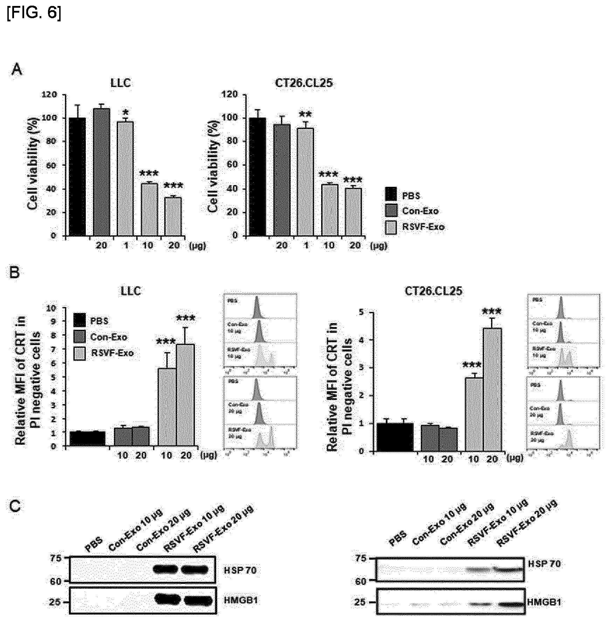

FIG. 6 shows immunogenic cell death of tumor cells by RSVF-Exo through endoplasmic reticulum (ER) stress. (A) Viability of RSVF-Exo-treated LLC and CT26.CL25 carcinoma cells. (B) Relative MFI of CRT expression in exosome-treated PI-negative CT26.CL25 and LLC cells. (C) Expression levels of HSP70 and HMGB1 released from exosome-treated CT26.CL25 cells.

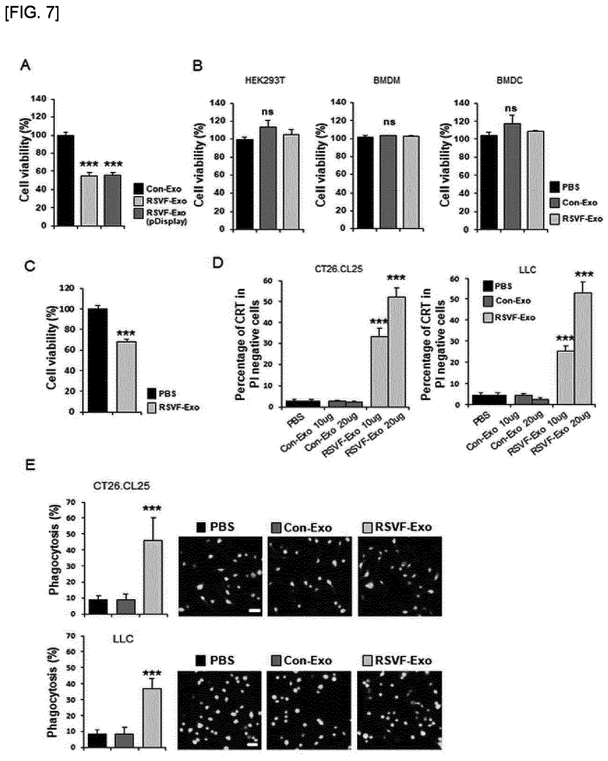

FIG. 7(A) Viability of exosome-treated cancer cells. (B) Viability of exosome-treated HEK293T, BMDM, and BMDC cells. (C) Viability of LLC cells after fusion of LLC cells with RSVF-Exo. (D) Percentage of CRT expression in exosome-treated PI-negative CT26.CL25 and LLC cells. (E) Percentage of phagocytosis in exosome-treated negative CT26.CL25 and LLC cells.

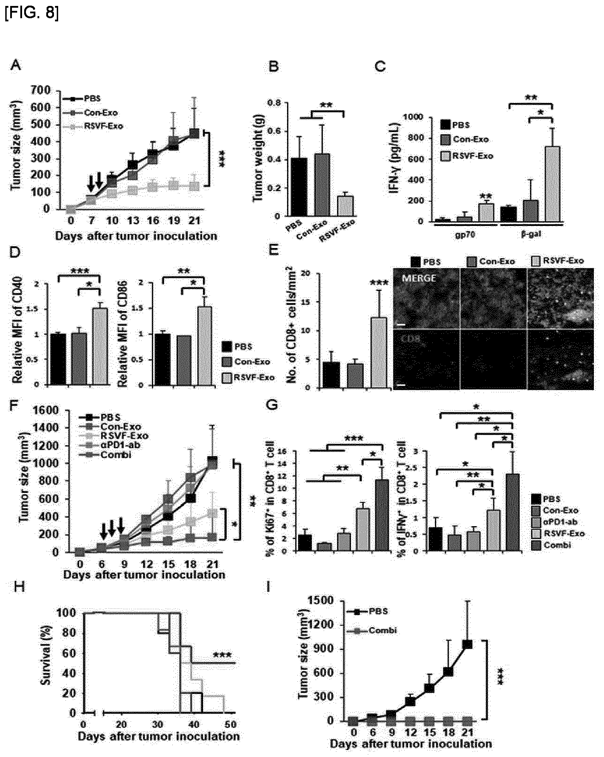

FIG. 8 shows the tumor growth inhibitory effect of RSVF-Exo in CT26.CL25 model mice. (A-E) CT26.CL25 tumor-bearing mice were intratumorally injected with 100 µg of RSVF-Exo, Con-Exo, or PBS every day a total of twice on days 7 and 8 after tumor inoculation (intratumoral injection; i.t.). (A) Mean tumor growth curve for 21 days (n = 10; *** p <0.001; ANOVA with Turkey's post-hoc test.). (B) Tumor weight on day 21 (n = 10; ** p <0.01; ANOVA with Turkey's post-hoc test.). (C) IFN (Interferon)-γ secretion against gp70 and β-galactose by single cells isolated from lymph node drainage (n = 3; * p <0.05, ** p <0.01; ANOVA with Turkey's post-hoc test.). (D) Relative MFI of CD40 and CD86 (n = 3-4; * p <0.05, ** p <0.01, *** p <0.001; ANOVA with Turkey's post-hoc test.). (E) The number of tumor-infiltrating CD8+ T cells counted from fluorescence microscopy images (right) (n = 3; *** p <0.001; ANOVA with Turkey's post-hoc test.) (left). (F-I) CT26.CL25 tumor-bearing mice were injected with 100 µg of RSVF-Exo, Con-Exo, or PBS every day a total of three times on days 6 to 8 after tumor inoculation, and 200 µg of anti-PD-1 antibody was injected on days 11, 13, 15, and 17. (F) Results of measuring tumor size after co-administration with PD1 antibody. (G) Survival rate of mice after co-administration with PD1 antibody. (H) Tumor growth curve when re-inoculating the same carcinoma to mice showing complete tumor remission, whose cancer completely disappeared after the corresponding treatment (n=2).

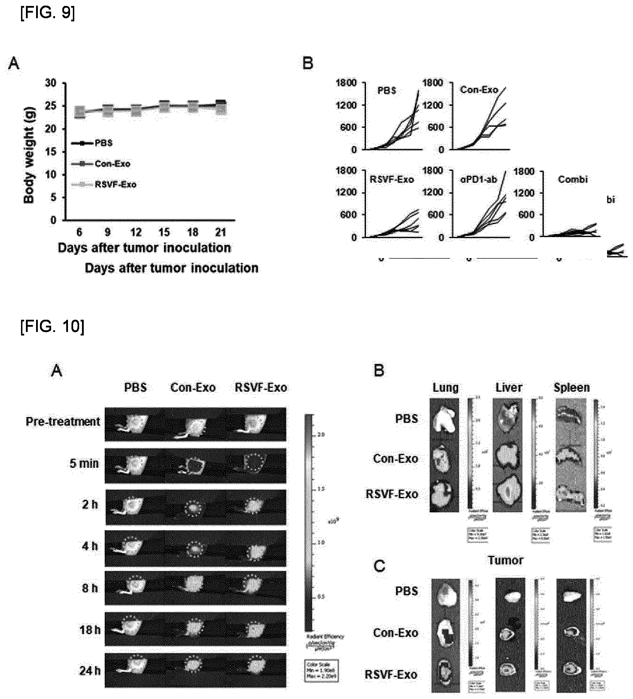

FIG. 9A shows the body weight of exosome-treated, tumor-bearing mice. FIG. 9B shows tumor growth curves for respective individuals in the f experimental group of FIG. 8.

FIG. 10 shows biodistribution of RSVF-Exo in CT26.CL25 tumor-bearing mice. (A) Results of visualizing biodistribution of exosomes at specific times (5 minutes, 2 hours, 4 hours, 8 hours, 18 hours, 24 hours) after intravenous injection of 300 µg of cyanine 5.5 NHS ester (Cy5.5-NHS)-labeled RSVF-Exo, Con-Exo, or PBS into CT26.CL25-bearing BALB/c nude mice. The orange dotted line indicates the tumor site. (B-C) The amount of Cy5.5-NHS signals in mouse-derived lung, liver, spleen, and tumor, 24 hours after injection.

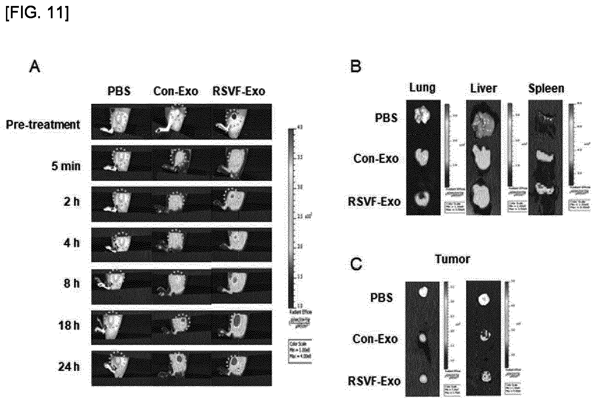

FIG. 11 shows biodistribution of RSVF-Exo in LLC tumor-bearing mice. (A) Results of visualizing biodistribution of exosomes at specific times (5 minutes, 2 hours, 4 hours, 8 hours, 18 hours, 24 hours) after intravenous injection of 300 µg of cyanine 5.5 NHS ester (Cy5.5-NHS)-labeled RSVF-Exo, Con-Exo, or PBS into LLC-bearing BALB/c nude mice. The orange dotted line indicates the tumor site. (B-C) The amount of Cy5.5-NHS signals in mouse-derived lung, liver, spleen, and tumor, 24 hours after injection.

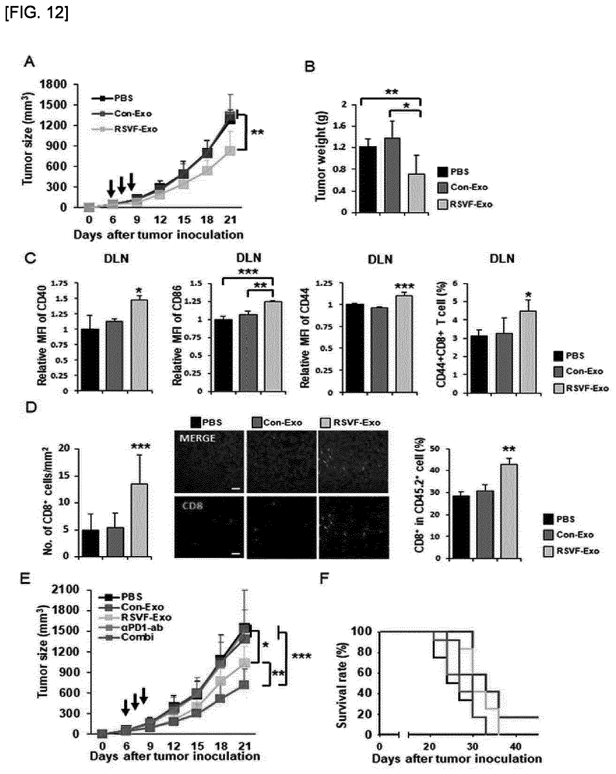

FIG. 12 shows the anti-tumor effect of systemically administered RSVF-Exo. (A-D) CT26.CL25 tumor-bearing mice were intravenously injected with 100 µg of RSVF-Exo, Con-Exo, or PBS a total of three times a day on days 6, 7, and 8 after tumor inoculation. (A) Mean tumor growth curve for 21 days (n = 8; ** p <0.01; ANOVA with Turkey's post-hoc test.). (B) Tumor weight on day 21 (n = 8; * p <0.05, ** p <0.01; ANOVA with Turkey's post-hoc test.). (C) Relative MFI of CD40 and CD86 of CD11C+ dendritic cells derived from lymph node drainage, and relative MFI of CD44 of CD45.2+CD3+CD8+ T cells (n = 3; * p <0.05, ** p <0.01, *** p <0.001; ANOVA with Turkey's post-hoc test.) The right graph shows a percentage of CD44+ CD8+ T cells (n = 5-6; ** p <0.01, *** p <0.001; ANOVA with Turkey's post-hoc test) (D) Fluorescence microscopy images and number of tumor-infiltrating CD8+ T cells counted from fluorescence images and percentage of CD8+ CD45.2+ cells (n = 3; ** p <0.01, *** p <0.001; ANOVA with Turkey's post-hoc test.). (E-F) CT26.CL25 tumor-bearing mice were intravenously injected with 150 µg of RSVF-Exo, Con-Exo, or PBS three times a day on days 6, 7, and 8 after tumor inoculation. Intraperitoneal injection (i.p.) was performed with 200 µg of anti-PD-1 antibody every other day a total of 4 times from day 11 after tumor inoculation. (E) Mean tumor growth curves compared for 21 days (n = 8; ** p <0.01; two-tailed unpaired student's t-test.). (F) Survival rate of mice after a combination therapy with immune checkpoint blockade.

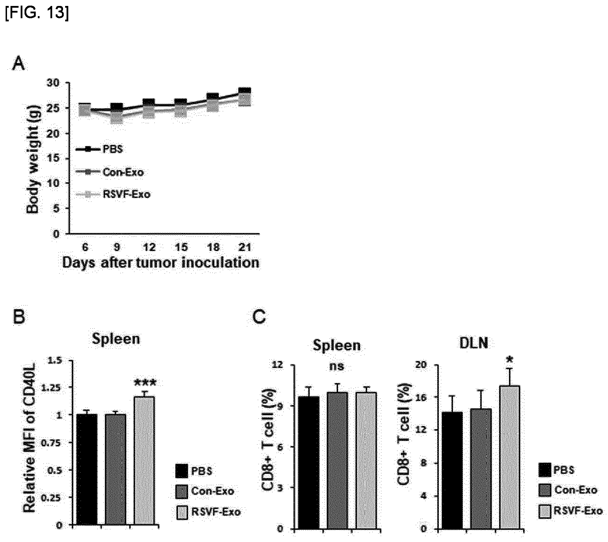

FIG. 13 shows the anti-tumor effect of systemically administered RSVF-Exo. (A) Body weight of exosome-treated, tumor-bearing mice. (B) Relative MFI of CD40 on dendritic cells in spleens derived from exosome-treated, tumor-bearing mice. (C) Percentage of CD8+ T cells in spleen and lymph node drainage.

### [Best Mode for Carrying Out Invention]

The present invention will be described in detail as follows. Meanwhile, each description and embodiment disclosed in this disclosure may also be applied to other descriptions and embodiments. That is, all combinations of various elements disclosed in this disclosure fall within the scope of the present invention. Further, the scope of the present invention is not limited by the specific description described below.

To achieve the above object, one aspect of the present invention provides an exosome (RSVF-Exo) expressing a respiratory syncytial virus F protein (RSVF).

As used herein, the term "respiratory syncytial virus (RSV)" belongs to the *Paramyxoviridae* family, and its genome consists of a single-stranded, negative-sense RNA molecule encoding 11 proteins, including 9 structural proteins (3 glycoproteins and 6 internal proteins) and 2 non-structural proteins. RSV is a virus that causes colds, bronchitis, pneumonia, and bronchiolitis in children and adults, and is known to be the most common cause of pneumonia in children under 5 years of age. Among the proteins constituting RSV, two major glycoproteins on the surface of RSV virions, an attachment glycoprotein (G) targeting ciliated cells of the airways, and a fusion (F) glycoprotein that causes the virion membrane to fuse with the target cell membrane control the early stages of infection. RSV is transmitted by inhalation of respiratory secretions and oral contact, and the incubation period is 4-5 days.

As used herein, the term "respiratory syncytial virus F protein (RSVF)" is a fusion (F) glycoprotein that causes the RSV virion membrane to fuse with the target cell membrane, and exists as a trimer in the virion membrane.

A gene encoding the F protein encodes a type I integral membrane protein that is synthesized as a 574 amino acid inactive precursor, F0, decorated with 5 to 6 N-linked glycans, depending on the strain. Three F0 monomers assemble into a trimer, and as the trimer passes through the Golgi, the monomers are activated by a furin-like host protease. The protease cleaves twice, after amino acids 109 and 136, to produce three polypeptides. The N-terminal and C-terminal cleavage products are F2 and F1 subunits, and are covalently linked to each other by two disulfide bonds. The F2 subunit contains two N-linked glycans, whereas the larger F1 subunit contains a single N-linked site. This F1 glycan is known to be essential for the protein to cause membrane fusion (Jason S. McLellan et al., Structure and Function of RSV Surface Glycoproteins, Curr Top Microbiol Immunol. 2013; 372: 83-104.).

As used herein, the term "exosome" refers to a small membrane-structured vesicle with a size of 50 nm to 200 nm, which contains proteins, DNA, RNA, etc., and is secreted out of cells in order to transmit signals between cells. Exosomes originate from specific intracellular compartments called multivesicular bodies (MVBs), are released and secreted out of cells, and are secreted from most cell types. Exosomes contain various proteins, DNA, RNA, etc. in cells. Substances which are contained in these exosomes and extracellularly secreted are re-introduced into other cells by fusion with cell membranes or endocytosis, and also serve as cell-cell communicators.

Considering that exosomes participate in cell-to-cell signaling and have a long circulation time in the blood without toxicity, they may be used as effective cell carriers. Exosomes are attracting much attention as effective therapeutic drug delivery carriers and may exhibit significant effects on cancer treatment. In addition, exosomes are known to be suitable for delivering biologically active membrane proteins because they have a phospholipid bilayer.

In one specific embodiment of the present invention, it was confirmed that the exosomes prepared according to the present invention express RSVF on the membrane (FIGS. 1C, 2A, and 2F), and have a circular form with a diameter of about 100 nm (FIGS. 1B and 2B).

The exosomes may specifically fuse with the membrane of cancer cells, but are not limited thereto.

In one specific embodiment of the present invention, it was examined whether the exosomes prepared according to the present invention fused with cell membranes of Lewis lung carcinoma (LLC) and CT26.CL25 colon carcinoma cell line, which express high levels of nucleolin, under *in vitro* conditions, and as a result, RSVF was delivered from exosomes to LLC and CT26.CL25 cell membranes (FIGS. 1E and 3A), and the membrane fusion activity was reduced by pre-blocking with an anti-nucleolin antibody (FIG. 1G), indicating that the membrane fusion activity depends on the interaction between RSVF and nucleolin. RSVF-Exo fused with cancer cell membranes in a concentration- and time-dependent manner, and remained the fusion with the cancer cell membrane for up to 4 hours (FIGS. 2C-2D and 2F). In contrast, in non-cancer cells expressing low levels of nucleolin, including HEK293T, BMDM, and BMDC, exosome-mediated RSVF fusion was rarely observed (FIGS. 1E and 3A).

It was also confirmed that RSVF was detected in tumor cells, not in bone marrow, endothelial and fibroblast cells, which are separated from mice treated with RSVF-Exo *in vivo* (FIGS. 1F and 3B).

These results indicate that RSVF-Exo preferentially fuses with cancer cells expressing high levels of nucleolin both *in vitro* and *in vivo.*

The exosomes may kill cancer cells through immunogenic cell death (ICD), but are not limited thereto.

RSVF is known to induce apoptosis by activating three endoplasmic reticulum (ER) stress sensor (inositol-requiring enzyme 1 (IRE1), protein kinase R-like endoplasmic reticulum kinase (PERK), and activating transcription factor 6 (ATF6), and prolonged ER stress is known to induce cell death through apoptosis and necrosis.

In other words, the RSVF-expressing exosomes (RSVF-Exo) of the present invention, which express the above-described RSVF on the surface, may preferentially fuse with malignant cells in a nucleolin-dependent manner, the nucleolin which is a major RSVF receptor and expressed at high levels on the surface of tumor cells, and may further induce ICD in an endoplasmic reticulum stress-mediated manner.

It is expected that the reason why the ICD is induced by RSVF-Exo of the present invention is that when RSVF-expressing cells are treated with bafilomycin A1, which is an antibiotic having vacuolar type H+ ATPase activity, fusion between phagosome-lysosome is inhibited to cause unfolded protein accumulation in exosomes, and as a result, a large amount of unfolded-proteins is accumulated inside RSVF-Exo derived from the cells. RSVF-Exo containing the unfolded-proteins fuses to the membrane of tumor cells to deliver the unfolded-proteins, and accordingly, the unfolded-proteins in the tumor cells increase, and the homeostasis control of the unfolded-protein response of the tumor cells fails to cause ER stress, leading to induction of ICD.

In other words, inside the exosomes according to the present invention, unfolded-proteins are accumulated due to inhibition of phagosome-lysosome fusion by treatment with a phagosome-lysosome fusion inhibitor.

The phagosome-lysosome fusion inhibition by the phagosome-lysosome fusion inhibitor may be induced by any one selected from the group consisting of promotion of vacuolar type H+ ATPase, inhibition of proteasome function, inhibition of autophagosome formation, and deacetylase inhibition, but is not limited thereto.

In one embodiment of the present invention, it was examined whether RSVF-Exo is able to induce ER stress, and as a result, it was confirmed that ATF4 and CHOP which are ER stress markers were up-regulated in the RSVF-Exo-treated groups (FIG. 4E).

RSVF-Exo-treated LLC and CT26.CL25 carcinoma cells showed low cell viability in a concentration-dependent manner, as compared to a Con-Exo-treated control group. However, RSVF-Exo did not induce cell death in normal cells such as HEK293T, BMDM, and BMDC (FIGS. 6A and 7B).

These results indicate that RSVF-Exo exhibits the cell killing effect only on cancer cells.

It was examined whether ER stress induces immunogenic cell death (ICD) in carcinoma cells, and as a result, RSVF-Exo increased calreticulin (CRT) expression on the cell surface, and increased the amount of ICD markers such as HSP70 (heat shock protein 70) and HMGB1 (high mobility group box 1) released from LLC and CT26.CL25 cells (FIGS. 6B to 6C and FIG. 7D).

Further, when RSVF-Exo is fused with tumors, viral proteins are expressed on the surface, which are easily recognized by the immune system to increase phagocytosis.

In one embodiment of the present invention, RSVF-Exo-treated LLC and CT26.CL25 carcinoma cells showed remarkably high phagocytosis, as compared to the Con-Exo-treated control group (FIG. 7E).

These results indicate that RSVF-Exo induces cancer cell death as well as activates the host immune system against cancer.

The exosomes may be accumulated in a tumor-specific manner to inhibit tumor growth, but are not limited thereto.

In one embodiment of the present invention, *in vivo* tumor-inhibitory effect of RSVF-Exo was examined using CT26.CL25 tumor-bearing mice, and as a result, when intratumoral treatment with RSVF-Exo showed about 70% recovery effect on tumor size (FIGS. 8A to 8B), and there was no significant difference in the body weight (FIG. 9A).

It is known that RSVF, which is a viral antigen, interacts with toll-like receptor 4 (TLR4) and activates downstream signaling pathways, which activate dendritic cells (DC) essential for T cell priming. Thus, it was examined whether RSVF-Exo is able to induce tumor-specific immunity, and as a result, in TDLN separated from RSVF-Exo-treated mice, the amount of IFN-γ which is secreted against a tumor-specific peptide gp70 and β-galactose was increased, as compared to the control group (FIG. 8C), and in the RSVF-Exo group, the expression levels of CD40 and CD86 which are dendritic cell maturation markers in CD11 c+ cells were increased (FIG. 8D).

Further, CD8+ T cell infiltration was analyzed in the CT26.CL25 tumor bearing BALB/c mouse-derived tumor tissue, and as a result, the amount of CD8+ T cells was increased in the tumor tissue of the RSVF-Exo-treated group (FIG. 8E).

These results indicate that RSVF-Exo may inhibit tumor growth *in vivo* by activating the anti-tumor immune response.

Meanwhile, oncolytic virus (OV) therapy does not affect healthy cells but adversely affects malignant cells, and has shown antitumor effects against various cancers, but depending on their origin, they are neutralized by antibodies and the innate immune system and rapidly eliminated from the blood. For this reason, there are problems in that OVs are difficult to apply to systemic treatment, and they can be applied only to limited tumors where intratumoral injection is easy, such as melanoma and head and neck cancer.

In contrast, systemic injection of RSVF-Exo of the present invention induces up-regulation of the immune system to exhibit an anti-tumor effect.

In one embodiment of the present invention, whether or not RSVF-Exo accumulates at the tumor site even after intravenous injection was examined in the CT26,CL25 tumor nude mouse model, and as a result, RSVF-Exo accumulated more at the tumor site than Con-Exo (FIGS. 10A and C), and compared to Con-Exo, there was no significant difference in the amount of Cy5.5-NHS signal in major organs such as lung, liver and spleen by targeting the tumor site (FIG. 10B). These results were also confirmed in the LLC model (FIGS. 11A to 11C).

It was examined whether or not RSVF-Exo is able to inhibit tumor growth *in vivo* through anti-tumor immunity when intravenously injected, and as a result, the RSVF-Exo treated group inhibited tumor growth more than the control group, and there was no difference in the body weight between groups (FIGS. 12A and 13A). It was also confirmed that CD40 and CD86, which are dendritic cell maturation markers, were upregulated in TDLN in the RSVF-Exo treated group (FIG. 12C).

As a result, RSVF-Exo treatment increased the frequency of CD8+ T cells in TDLN and an activation marker CD40 ligand (CD40L) on CD8+ T cells in the spleen (FIG. 12D, FIGS. 13B to 13C).

In addition, the expression and frequency of CD44 on CD8+ T cells were evaluated to determine whether RSVF-Exo promotes CD8+ T cell memory. As a result, RSVF-Exo effectively increased the proportion of antigen-experienced CD44 expressing CD8+ T cells in TDLN (FIG. 12C). In addition, the amount of tumor-infiltrating CD8+ T cells was significantly increased by RSVF-Exo (FIG. 12D).

These results show that even when injected intravenously, RSVF-Exo accumulates at the tumor site not only to suppress tumor growth *in vivo* through anti-tumor immunity, but also to promote the frequency and activation of CD8+ T cells and to promote CD8+ T cell memory, thereby remarkably increasing the amount of tumor-infiltrating CD8+ T cells.

Another aspect of the present invention provides a pharmaceutical composition for preventing or treating cancer, the pharmaceutical composition including the exosome as an active ingredient.

The terms used herein are the same as described above.

As used herein, the term "cancer" refers to a tumor formed from normal tissue cells by the action or cause of various physical, chemical, and biological carcinogenic substances, and the cancer includes any cancer without limitation to the type thereof, and examples thereof may include esophageal cancer, stomach cancer, colon cancer, rectal cancer, oral cancer, pharyngeal cancer, larynx cancer, lung cancer, colon cancer, breast cancer, cervical cancer, endometrial cancer, ovarian cancer, prostate cancer, testicular cancer, bladder cancer, kidney cancer, liver cancer, pancreatic cancer, bone cancer, connective tissue cancer, skin cancer, brain cancer, thyroid cancer, leukemia, Hodgkin's disease, lymphoma, or multiple myeloma blood cancer, specifically, colon cancer or lung cancer, but are not limited thereto.

The pharmaceutical composition of the present invention has use in the "prevention" and/or "treatment" of cancer. With regard to the use in the prevention, the pharmaceutical composition of the present invention may be administered to an individual having the disease or symptom described in the present invention or suspected of being at the risk of having the disease. With regard to the use in the treatment, the pharmaceutical composition of the present invention may be administered in an amount sufficient to an individual such as a patient already having the disease described in the present invention in order to treat or at least partially arrest the disease. The effective amount for this use will depend on the severity and course of the disease or symptoms, previous treatment, individual's health conditions and responsiveness to medications, and a physician or veterinarian's judgment.

The pharmaceutical composition of the present invention may further include an appropriate carrier, excipient, or diluent that is commonly used. In this regard, the content of the active ingredient RSVF-Exo included in the composition is not particularly limited, but the active ingredient RSVF-Exo may be included in an amount of 0.0001% by weight to 10% by weight, preferably 0.001% by weight to 1% by weight with respect to the total weight of the composition.

The pharmaceutical composition may have any one formulation selected from the group consisting of tablets, pills, powders, granules, capsules, suspensions, solutions for internal use, emulsions, syrups, sterile aqueous solutions, non-aqueous solvents, suspensions, emulsions, lyophilized preparations, and suppositories, and may be of various oral or parenteral formulations. When formulated, the formulation may be prepared using a diluent or an excipient, such as a filler, an extender, a binder, a wetting agent, a disintegrant, a surfactant, etc. which are commonly used. Solid formulations for oral administration include tablets, pills, powders, granules, capsules, etc., which may be prepared by blending one or more compounds with at least one excipient, for example, starch, calcium carbonate, sucrose or lactose, gelatin, etc. In addition to the simple excipient, a lubricant such as magnesium stearate, talc, etc., may also be used. Liquid formulations for oral administration include suspensions, solutions for internal use, emulsions, syrups, etc., and in addition to water and liquid paraffins which are simple diluents commonly used, various excipients, for example, wetting agents, sweetening agents, fragrances, preservatives, etc. may be included. Formulations for parenteral administration include sterile aqueous solutions, non-aqueous solvents, suspensions, emulsions, lyophilized formulations, and suppositories. Propylene glycol, polyethylene glycol, vegetable oils such as olive oil, injectable esters such as ethyl oleate, etc. may be used as the non-aqueous solvents and suspensions. As the base for suppositories, witepsol, macrogol, tween 61, cacao butter, laurin butter, glycerogelatin, etc. may be used.

The composition of the present invention may be administered to an individual in a pharmaceutically effective amount.

As used herein, the term "pharmaceutically effective amount" refers to an amount sufficient to treat diseases, at a reasonable benefit/risk ratio applicable to any medical treatment. The effective dosage level may be determined depending on factors including kind of the individual and severity, age, sex, kind of the disease, activity of the drug, sensitivity to the drug, administration time, administration route and excretion rate, duration of treatment, drugs used simultaneously, and other factors known in the medical field.

The pharmaceutical composition of the present invention may be administered alone or in combination with other therapeutic agents, and may be administered sequentially or simultaneously with existing therapeutic agents. The composition may be administered in a single or multiple dosage form.

The RSVF-Exo of the present invention synergistically improved the efficacy of immune checkpoint blockade when applied together with an immune checkpoint blockade therapy.

In one embodiment of the present invention, to examine RSVF-Exo-induced, CD8+ T cell immunity that increases the efficacy of the immune checkpoint blockade therapy, CT26.CL25 tumor-bearing BALB/c mice were subjected to a combination therapy of RSVF-Exo and anti-PD-1 blockade. As a result, an anti-PD-1 antibody monotherapy group and a Con-Exo or PBS-administered control group showed no tumor suppression effect, whereas the combination therapy group of RSVF-Exo and anti-PD-1 antibody showed a synergistic effect on tumor suppression and improved the survival rate of mice (FIGS. 8F to 8I, and FIGS. 12E to 12F).

These results indicate that systemic administration of RSVF-Exo via intravenous injection may suppress tumor growth without toxicity, unlike oncolytic virus, and may enhance the therapeutic effect of immune checkpoint blockade.

It is important to administer the pharmaceutical composition in the minimum amount that may exhibit the maximum effect without causing side effects, considering all the above-described factors, and those skilled in the art may readily determine. The preferred dosage of the composition of the present invention varies depending on a patient's conditions and body weight, severity of the disease, the form of the drug, the administration route and duration, and administration may be made once a day or divided several times.

The pharmaceutical composition is not particularly limited as long as it may be administered to an individual for the purpose of preventing or treating cancer. A mode of the administration includes any method without limitation as long as it is a common method in the art. The pharmaceutical composition may be administered parenterally, subcutaneously, intraperitoneally, intrapulmonary and intranasally, and for local treatment, if necessary, by any suitable method including intralesional administration. For example, it may be administered by oral, intraperitoneal, rectal or intravenous, intramuscular, subcutaneous, intrauterine or intracerebral injection, but is not limited thereto.

The pharmaceutical composition of the present invention may be generally administered at a concentration of 0.001 µg/kg to 1,000 µg/kg, specifically, at a concentration of 0.05 µg/kg to 1,000 µg/kg, and more specifically, at a concentration of 1 µg/kg to 100 µg/kg, but is not limited thereto.

Still another aspect of the present invention provides a method of treating cancer, the method including the step of administering the pharmaceutical composition to an individual.

The terms used herein are the same as described above.

As used herein, the term "individual" refers to all animals that have or may have cancer, and the individual may be effectively treated by administering the pharmaceutical composition of the present invention to the individual suspected of having cancer. The individual is not particularly limited, and may include, for example, animals such as monkeys, dogs, cats, rabbits, guinea pigs, rats, mice, cows, sheep, pigs, goats, etc., birds, and fish, etc., but is not limited thereto.

As used herein, the term "administering" means introduction of the pharmaceutical composition of the present invention into an individual suspected of having cancer by a certain appropriate method. As long as it reaches target tissues, any route, whether oral or parenteral, may be taken for administering. The pharmaceutical composition of the present invention may be administered in a pharmaceutically effective amount, and the pharmaceutically effective amount is the same as described above.

An appropriate total daily dose may be determined through appropriate medical judgment by a physician, and generally, administration may be made at a concentration of 0.001 µg/kg to 1,000 µg/kg, specifically, at a concentration of 0.05 µg/kg to 1,000 µg/kg, and more specifically, at a concentration of 1 µg/kg to 100 µg/kg once a day or divided several times.

Still another aspect of the present invention provides a vaccine composition for preventing cancer, the vaccine composition including the exosome as an active ingredient.

The terms used herein are the same as described above.

As used herein, the term "vaccine" refers to inducing a cellular immune response in an individual for the purpose of preventing or treating cancer. The vaccine composition according to the present invention may induce cellular immunity against various cancer cells.

The vaccine composition may additionally include one or more selected from the group consisting of a solvent, an adjuvant, and an excipient. As the solvent, physiological saline or distilled water may be used. As the adjuvant, Freund's incomplete or complete adjuvant, aluminum hydroxide gel, and vegetable and mineral oil may be used. As the excipient, aluminum phosphate, aluminum hydroxide, or aluminum potassium sulfate may be used. In addition, a known material used in vaccine production may be further included.

The vaccine composition may be prepared as an oral or parenteral formulation, and is preferably prepared as a solution for injection, which is a parenteral formulation, and may be administered through an intradermal, intramuscular, intraperitoneal, intravenous, subcutaneous, nasal, or epidural route.

Still another object of the present invention provides a kit for preventing or treating cancer, the kit including a first composition including the exosome expressing the respiratory syncytial virus F protein; and a second composition including an anticancer agent as an active ingredient.

The terms used herein are the same as described above.

The administration route and frequency of the first composition and the second composition may be independent of each other, but are not limited thereto.

The anticancer agent may be used without limitation as long as it is known in the art, and may be, for example, an anticancer agent used in an immune checkpoint blockade therapy, specifically, an anti-PD-1 antibody, but is not limited thereto.

The kit of the present invention refers to a tool that may be used for preventing or treating cancer by including the first composition and the second composition, respectively. The type of the kit is not particularly limited, and any type of kit commonly used in the art may be used.

The kit of the present invention may be packaged in a form in which the first composition and the second composition are contained in individual containers, or in a form in which they are contained in one container divided into one or more compartments, and the first composition and the second composition may be packaged in a unit dosage form for one administration dose.

The first composition and the second composition in the kit may be individually administered in combination at an appropriate time according to health conditions of an individual to be administered. In other words, the administration route and frequency of the first composition and the second composition may be independent of each other.

The kit of the present invention may further include an instruction manual describing the dosage, administration method, and administration frequency of each of the first composition and the second composition.

Still another aspect of the present invention provides a method of preventing or treating cancer using the kit.

The terms used herein are the same as described above.

Still another object of the present invention provides a method of preparing the exosome expressing the respiratory syncytial virus F protein, the method including the steps of i) transfecting cells with a plasmid including a polynucleotide encoding the respiratory syncytial virus F protein (RSVF); ii) treating the cells of the step i) with a drug; and iii) separating the exosome from the supernatant of the cells of the step ii).

The terms used herein are the same as described above.

The step i) may include the step of transfecting cells with a plasmid including a polynucleotide encoding the respiratory RSVF.

In one embodiment of the present invention, a plasmid was prepared, in which a polynucleotide (SEQ ID NO: 2) encoding RSVF(Respiratory syncytial virus fusion protein) was included in a pCMV6-Entry(PS100001) vector (origene). The prepared plasmid was transfected into human embryonic kidney 293T (HEK293T) cells using lipofectamine 3000, but is not limited thereto.

The step ii) may include the step of treating the cells of the step i) with a drug. Specifically, cells producing exosomes, in which unfolded proteins are accumulated, may be prepared by treating the cells with a drug.

The drug may be any one or more selected from the group consisting of a phagosome-lysosome fusion inhibitor, a proteasome function inhibitor, and an autophagosome function inhibitor, but any drug may be used without limitation, as long as it induces cells to produce exosomes, in which unfolded proteins are accumulated. Specifically, the phagosome-lysosome fusion inhibitor may be, for example, bafilomycin A1, chloroquine, hydroxychloroquine, quinacrine, Monensin, E64d, or pepstatin A. The proteasome function inhibitor may be, for example, bortezomib or carfilzomib. The autophagosome function inhibitor may be, for example, verteporfin or vinca alkaloids, but is not limited thereto.

Th step ii) may be performed 24 hours after the cells of the step i) are transfected, but is not limited thereto.

In one embodiment of the present invention, 24 hours after the cells in the step i) were transfected, the cells were treated with *Streptomyces* griseus-derived bafilomycin A1 (BafA1) dissolved in dimethyl sulfoxide (DMSO) at a concentration of 10 nM to 50 nM, but is not limited thereto.

The step iii) may include the step of separating the exosome from the supernatant of the cells of the step ii).

The step iii) may be performed 48 hours after the cells of the step i) are transfected, i.e., 48 hours after bafilomycin A1 treatment, but is not limited thereto.

In one embodiment of the present invention, 48 hours after the cells of the step i) are transfected, the supernatant of HEK293T cells was harvested, centrifuged, and filtered to separate the exosomes, but is not limited thereto.

According to the above method, when RSVF-expressing exosomes are prepared by treating RSVF-expressing cells with bafilomycin A1, which is an antibiotic having vacuolar type H+ ATPase activity, the prepared RSVF-expressing exosomes inhibit phagosome-lysosome fusion as described above to cause accumulation of unfolded-proteins, etc., and accordingly, as a large amount of the unfolded-proteins accumulates inside RSVF-Exo derived from the cells, ICD is induced, thereby exhibiting cancer cell killing and tumor growth inhibitory effects.

Still another aspect of the present invention provides use of the exosome in the prevention or treatment of cancer.

### [Mode for Carrying Out Invention]

Hereinafter, the present invention will be described in detail in light of exemplary embodiments for better understanding of the present invention. However, the following exemplary embodiments are provided only for illustrating the content of the present invention, but the scope of the present invention is not limited to the following exemplary embodiment. The exemplary embodiments of the present invention are provided such that this disclosure will be thorough and complete and will fully convey the concept of the present invention to those skilled in the art.

### Example 1. Preparation of respiratory syncytial virus F protein (RSVF)-expressing exosome (RSVF-Exo)

Human embryonic kidney 293T (HEK293T) cells were cultured in a DMEM medium (Dulbecco's Modified Eagle Medium) supplemented with 10% fetal bovine serum (FBS) and 1% antibiotics (Antibiotic-Antimycotic, Gibco) under conditions of 37°C and 5% CO₂.

In addition, a plasmid containing a polynucleotide (SEQ ID NO: 2) encoding RSVF (Respiratory syncytial virus fusion protein) in a pCMV6-Entry (PS100001) vector (origene) which was treated with restriction enzymes Sgfl and Mlul was prepared.

When confluency of HEK293T cells reached 90%, the medium was replaced with a DMEM containing 1% glutamax and 1% antibiotics. 2 hours later, HEK293T cells were transfected with the plasmid containing the polynucleotide encoding RSVF using lipofectamine 3000 (Invitrogen). 24 hours later, the cells were treated with *Streptomyces* griseus-derived bafilomycin A1 (BafA1) dissolved in dimethyl sulfoxide (DMSO) at different concentrations (10 nM, 30 nM, 50 nM). For exosome isolation, supernatants from HEK293T cells were harvested 48 hours after transfection. To remove microvesicles and cell debris, the supernatants were centrifuged at 300 g for 10 minutes, at 2000 g for 10 minutes, and at 10,000 g for 30 minutes. The supernatants were filtered through a 0.22 µm fine pore filter, and centrifuged at 150,000 g for 3 hours to obtain exosome pellets. The exosome pellets were resuspended in PBS (phosphate-buffered saline) containing a proteinase inhibitor cocktail (PIC, Roche), and preserved at 4 °C to prepare RSVF-Exo.

As a control, a control exosome (Con-Exo) was prepared by harvesting the supernatant of a medium, in which the HEK293T cells transfected with the empty plasmid were cultured.

### Example 2. Characterization of RSVF-Exo

The total amount of proteins in RSVF-Exo prepared in Example 1 was measured using a bicinchoninic acid (BCA) protein assay. To dissolve the exosomes, an equal amount of exosome protein was added to RIPA (Radioimmunoprecipitation assay) buffer and mixed with an SDS-PAGE (sodium dodecyl sulphate-polyacrylamide gel electrophoresis) buffer. After gel electrophoresis, bands were transferred to a nitrocellulose membrane. After pre-blocking with 5% skim milk dissolved in TBST (Tris Buffered Saline)-added tween-20 at room temperature for 1 hour, the membrane was incubated with a primary antibody at 4°C overnight. To identify exosome markers and FLAG (Sigma-Aldrich, 1 : 500) for detection of RSVF expression, Tsg101 (Santa Cruz, 1 : 500), Alix (Santa Cruz, 1 : 500), and CD81 (Santa Cruz, 1 : 500) were treated. The membrane was incubated with an HRP (horseradish peroxidase)-conjugated anti-mouse (Sigma-Aldrich, 1 : 3000) or anti-rabbit (Sigma-Aldrich, 1 : 3000) secondary antibody, and then analyzed using an imaging system (ChemiDoc Imaging System, Bio-Rad).

The amount of RSVF present on the RSVF-Exo membrane was analyzed using flow cytometry. Aldehyde/sulfate latex beads were mixed with exosomes, diluted in PBS, and incubated at 4°C overnight. The exosome-conjugated latex beads were pre-blocked for 30 minutes using a 1 M glycine buffer, washed with 0.5% bovine serum albumin, and dissolved in PBS. The beads were reacted with an RSVF antibody (Abeam, 1:100) at 4°C for 1 hour, and then treated with an Alexa Fluor 488-conjugated secondary antibody (Jackson ImmunoResearch, 1:200) at room temperature for 1 hour. After removing the unconjugated antibody by centrifugation, the beads were analyzed by flow cytometry (BD Biosciences, Accuri C6).

The size of RSVF-Exo was measured by Zetasizer (Malvern Panalytical, Nano ZS). 1 µg of RSVF-exosomes were diluted in 500 µl of PBS and placed in a semi-micro cuvette (ratiolab). The size distribution of exosomes was analyzed at a fixed angle of 173° and 25°C.

In addition, the structure of RSVF-Exo was identified through a cryogenic transmission electron microscope (cryo-TEM; Tecnai system). RSVF-Exo was placed on a thin carbon film covered with a copper grid, and vitrified by Vitrobot (FEI). The vitrified sample was maintained in liquid nitrogen until sample images were taken.

As a result, it was confirmed that the purified RSVF-Exo expressed RSVF on the membrane (FIG. 2A), and the membrane was circular (FIGS. 2A and 2B) with a diameter of about 100 nm (FIG. 1B).

### Example 3. Membrane fusion of RSVF-Exo and cancer cells

### 3-1. In vitro

Malignant cells express high levels of nucleolin, which is a major receptor for RSVF. To examine whether RSVF-Exo is able to fuse with the membrane of cancer cells *in vitro,* RSVF protein expression was analyzed in Lewis lung carcinoma (LLC) and CT26.CL25 colon carcinoma cell lines.

LLC cells were cultured in a DMEM medium (Dulbecco's Modified Eagle Medium) supplemented with 10% fetal bovine serum and 1% antibiotics (Antibiotic-Antimycotic, Gibco, 15240-062) under conditions of 37°C and 5% CO₂. CT26.CL25 colon carcinoma cells, bone marrow derived macrophages (BMDMs), and bone marrow derived dendritic cells (BMDCs) were cultured in an RPMI 1640 medium (Roswell Park Memorial Institute 1640) supplemented with 10% FBS and 1% antibiotics under conditions of 37°C and 5% CO₂.

The BMDMs were differentiated by treating C57BL/6 mouse-derived bone marrow cells with 20 ng/ml of M-CSF (PeproTech, 315-02) on days 1, 2, and 3, and then by replacing the medium with a fresh medium containing 20 ng/ml of M-CSF on days 4 and 6. In addition, the BMDCs were differentiated by treating C57BL/6 mouse-derived bone marrow cells with 200 ng/ml of Flt3-Ligand (PeproTech, 250-31L) and 0.1% β-mercaptoethanol for 10 days.

To determine the amount of nucleolin present on the LLC, CT26.CL25, HEK293T, BMDC, and BMDM cell membranes, 5 × 10⁵ cells were fixed with 0.25% paraformaldehyde at 4°C for 1 hour, respectively. The cells were pre-blocked with 3% BSA (Bovine serum albumin), dissolved in PBS at 4°C for 15 minutes, and treated with a nucleolin antibody (Abeam, 1:200) at 4°C for 45 minutes. After washing with DPBS (Dulbecco's Phosphate-Buffered Saline), samples were treated with an Alexa Fluor 647-conjugated secondary antibody (Jackson Immuno Research, 1:400) at 4°C for 30 minutes. After washing the cells with DPBS, the amount of nucleolin on the cell surface was measured using flow cytometry.

In addition, 3.75 × 10⁵ LLC or CT26.CL25 colon carcinoma cells were treated with 25 µg, 50 µg, or 100 µg of RSVF-Exo, Con-Exo, or PBS, respectively, and then incubated in a serum-free medium at 37°C for 5 minutes (or 1 hour, 4 hours). The exosomes were removed by centrifugation, and the cells were maintained in a serum-supplemented medium at 37°C for 1 hour for stabilization. After removing the medium, the cells were pre-blocked with 3% BSA at 4°C for 15 minutes, and incubated with an RSVF antibody (Abeam, 1:200) at 4°C for 45 minutes. To remove unconjugated antibodies, the cells were washed with DPBS, and then an Alexa Fluor 647-conjugated secondary antibody (1 : 400) was added to the sample and maintained at 4 °C for 30 minutes. The cells were washed with DPBS, and the amount of RSVF on the cell membrane was measured by flow cytometry.

For confocal microscopy analysis, 3.75 × 10⁵ CT26.CL25 cells were treated with 50 µg of RSVF-Exo, seeded in Nunc^{™} Lab-Tek^{™} (Thermo fisher Scientific), and incubated at 37°C for 1 hour, 4 hours, or 16 hours. Then, CT26.CL25 cells were fixed with 4% paraformaldehyde and pre-blocked with a 3% BSA solution. Subsequently, the plasma membrane of each cell was labeled with wheat germ agglutinin (WGA, 5 µg ml-1, Alexa FluorTM-633-conjugated dye, ThermoFisher) in a 1% BSA solution. RSVF proteins on the surface of each cell were labeled with an RSVF antibody (Abcam, 1:200) and an Alexa Fluor 488-conjugated secondary antibody.

To identify whether RSVF fuses with cells in a nucleolin-dependent manner, 3.75 × 10⁵ cells were fixed with 0.25% paraformaldehyde at 4°C for 1 hour. After removing paraformaldehyde, the fixed cells were treated with a nucleolin antibody (Santa Cruz, 1:100) and incubated at 37°C for 1 hour. Unconjugated nucleolin antibody was removed by centrifugation. The corresponding cells were incubated with 25 µg of RSVF exosomes for 5 minutes. Unconjugated RSVF exosomes were removed by centrifugation. For stabilization, cells were maintained in serum-free medium at 37°C for 1 hour. After removing the medium, the cells were pre-blocked with 3% BSA at 4°C for 15 minutes, and incubated with an RSVF antibody (Abcam, 1:200) at 4°C for 45 minutes. After washing with DPBS to remove unconjugated antibody, an Alexa Fluor 647-conjugated secondary antibody (1 : 400) was added to the sample and maintained at 4°C for 30 minutes. The cells were washed with DPBS, and the amount of RSVF on the cell membrane was measured by flow cytometry.

As a result, it was shown that RSVF was transferred from the exosomes to the membranes of LLC and CT26.CL25 cells highly expressing nucleolin (FIG. 1E and FIG. 3A).

In addition, the membrane fusion activity was reduced by pre-blocking with an anti-nucleolin antibody (FIG. 1G), indicating that the membrane fusion activity is dependent on the interaction between RSVF and nucleolin.

In non-cancer cells expressing low levels of nucleolin, including HEK293T, BMDM and BMDC, exosome-mediated RSVF fusion was hardly observed (FIG. 1E and FIG. 3A). In addition, RSVF-Exo fused with the cancer cell membrane in a concentration-dependent manner and bound to the cancer cell membrane for up to 4 hours (FIGS. 2C-2D).

### 3-2. In vivo

To examine whether RSVF-Exo is able to bind with cancer cells *in vivo,* 1 × 10⁶ CT26.CL25-mCherry cells were inoculated into the left flank of 8-week-old male BALB/c nude mice. When the average tumor volume reached 150 mm³, 100 µg of RSVF-Exo, Con-Exo, or PBS was injected intratumorally every day a total of twice on days 10 and 11 after tumor inoculation. 3 hours after the last injection, mice were sacrificed and tumors were extracted. After removing apoptotic cells using a kit (Dead Cell Removal Kit, Miltenyi Biotec), cells were sorted using CD45, CD31, and CD90.2 microbeads (MicroBead, Miltenyi Biotec). The amount of RSVF in tumors, CD45+, CD31+, and CD90.2+ cells was evaluated using flow cytometry.

As a result, RSVF was detected in tumor cells other than in bone marrow, endothelial, and fibroblast cells isolated from mice treated with RSVF-Exo *in vivo* (FIGS. 1F and 3B).

These results indicate that RSVF-Exo preferentially fuses with cancer cells expressing high levels of nucleolin both *in vitro* and *in vivo.*

### Example 4. Verification of aggresome delivery of RSVF-Exo to cancer cells

Flow cytometry was used to measure the amount of ubiquitinated proteins in RSVF-Exo prepared in Example 1. Aldehyde/sulfate latex beads were mixed with exosomes, diluted in PBS, and allowed to react at 4°C overnight. The exosome-conjugated latex beads were pre-blocked using a 1 M glycine buffer for 30 minutes, washed with 0.5% bovine serum albumin, and dissolved in PBS. The corresponding exosomes were reacted using a permeabilizing solution of a Proteostat^{®} Aggresome detection kit at 4°C for 30 minutes. Then, after washing with DPBS solution, the exosomes were pre-blocked using a 3% bovine serum albumin solution, and then, reacted using an aggresome detection reagent of a Proteostat^{®} Aggresome detection kit at room temperature for 30 minutes. Finally, the exosomes washed with a DPBS solution were analyzed by flow cytometry (FIG. 4A).

Flow cytometry was used to examine accumulation of the aggresomes present inside the RSVF-Exo prepared in Example 1 into cancer cells. Each of 5 × 10⁵ CT26.CL25 and LLC tumor cells and 100 µg of RSVF-Exo obtained with bafilomycin treatment and 140 µg of RSVF-Exo obtained without bafilomycin treatment were reacted in a DMEM medium (Dulbecco's Modified Eagle Medium) supplemented with 1% antibiotic (Antibiotic-Antimycotic, Gibco, 15240-062) at 37 °C for 2 hours. Thereafter, the cells were centrifuged at 3000 rpm for 3 minutes, and the pellet was washed with a DPBS solution, and then fixed with 4% paraformaldehyde at room temperature for 7 minutes. The cells were pre-blocked with 3% BSA (Bovine serum albumin) at room temperature for 15 minutes, and thereafter, reacted using an aggresome detection reagent of a Proteostat^{®} Aggresome detection kit at room temperature for 30 minutes. Finally, after washing with a DPBS solution, the cells were analyzed by flow cytometry (FIGS. 4B and 5B).

Confocal microscopy was used to examine accumulation of the aggresomes present inside RSVF-Exo prepared in Example 1 into cancer cells. 5 × 10⁵ CT26.CL25 cells were seeded in Nunc^{™} Lab-Tek^{™} (Thermo fisher Scientific), and then cultured at 37°C overnight, and next day, replaced with a DMEM (Dulbecco's Modified Eagle Medium) supplemented with 1% antibiotic (Antibiotic-Antimycotic, Gibco, 15240-062). Then, 100 µg of RSVF-Exo obtained with bafilomycin treatment and 140 µg of RSVF-Exo obtained without bafilomycin treatment were treated and reacted at 37 °C for 2 hours. After washing with DPBS, the cells were fixed with 4% paraformaldehyde at room temperature for 7 minutes. Then, after washing with a DPBS solution, the cells were reacted using a permeabilizing solution of a Proteostat^{®} Aggresome detection kit at 4 °C for 30 minutes. Subsequently, the cells were pre-blocked with 1% BSA (Bovine serum albumin) at room temperature for 15 minutes, washed with a DPBS solution, and then reacted using an aggresome detection reagent of a Proteostat^{®} Aggresome detection kit at room temperature for 30 minutes. Then, the aggresomes present inside the cancer cells were photographed using a confocal microscope (FIG. 4C).

The amount of aggresomes accumulated in RSVF-Exo-extracted mother cells increased according to treatment with different concentrations of bafilomycin A1 (10 nm, 30 nm, and 50 nm) (FIG. 5A).

### Example 5. Cancer cell death induction effect of RSVF-Exo

4 × 10⁵ CT26.CL25 cells were incubated in a 6-well plate at 37°C overnight. The medium was replaced with a serum-free medium containing 100 µg of RSVF-Exo, Con-Exo, or PBS, and maintained at 37°C for 24 hours. Next day, cells were lysed using RIPA buffer, and the amount of ER stress markers was analyzed by Western blotting. CHOP (C/EBP homologus protein, Santa Cruz, 1 : 200), ATF4 (activating transcription factor 4, Santa Cruz, 1 : 100), elF2α (eukaryotic translation initiation factor 2 alpha subunit, CST, 1 : 1000), Phospho-elF2α (CST, 1 : 1000), and GAPDH (Glyceraldehyde-3-Phosphate Dehydrogenase, R & D Systems, 1 : 2000) antibodies were applied to the membrane, followed by incubation at 4°C overnight. After washing the unconjugated antibody with TBST, the sample was treated with an HRP-conjugated secondary antibody at room temperature for 1 hour.

As a result, ATF4 and CHOP which are ER stress markers were upregulated in the RSVF-Exo-treated group (FIG. 4E).

To investigate whether RSVF-Exo is able to induce cell death, 2 × 10⁴ CT26.CL25 and LLC cells were treated with RSVF-Exo, Con-Exo, or PBS, and incubated in a serum-free medium at 37°C for 24 hours. After reacting 2 × 10⁴ LLC cells or CT26.CL25 cells with 200 µg of RSVF-Exo at 37 °C for 5 minutes, only cancer cells were obtained by centrifugation at 3000 rpm for 3 minutes. The corresponding cells were seeded in a 96-well plate and incubated in a serum-free medium at 37°C for 24 hours. Then, cell viability was measured using CCK (Cell Counting Kit)-8. Absorbance at 450 nm was measured using a microplate reader (Molecular Devices, SpectraMAX 340).

As a result, RSVF-Exo-treated LLC and CT26.CL25 cancer cells showed lower cell viability in a concentration-dependent manner, as compared to the Con-Exo-treated control group. However, RSVF-Exo did not induce cell death in normal cells such as HEK293T, BMDM, and BMDC (FIGS. 6A and 7B).

The above results suggest that RSVF-Exo exhibits the cell killing effect only on cancer cells.

To examine whether ER stress induces immunogenic cell death (ICD) in cancer cells, CT26.CL25 cells were treated with exosomes, and then the amounts of ICD markers such as calreticulin (CRT), HSP70 (heat shock protein 70), and HMGB1 (high mobility group box 1) were analyzed. To determine whether RSVF-Exo is able to induce damage-associated molecular pattern (DAMP) signals, 4 × 10⁵ CT26.CL25 and LLC cells were seeded in a 6-well plate, respectively and incubated at 37°C overnight. Then, the cells were treated with RSVF-Exo, Con-Exo, or PBS, and then maintained in a serum-depleted medium. 24 hours after treatment, the cells were isolated to detect surface expression of calreticulin. The supernatant was collected and concentrated to examine HMGB1 and HSP70.

In detail, for a CRT assay, 5 × 10⁵ CT26.CL25 cells treated with RSVF-Exo were fixed with 0.25% paraformaldehyde at 4°C for 5 minutes, then pre-blocked with 3% BSA, and then diluted in PBS at 4 °C for 15 minutes. Samples were treated with a calreticulin antibody (Abeam, 1 : 250) at 4°C for 45 minutes. After removing the unconjugated antibody, the cells were treated with an Alexa Fluor 488-conjugated secondary antibody (1:200), and maintained at 4°C for 30 minutes. The sample was washed using DPBS, and stained with propidium iodide (PI, Sigma-Aldrich, 1:200) at 4°C for 15 minutes to examine cell viability. The amount of calreticulin on the cell membrane was analyzed using flow cytometry.

For Western blot analysis of HSP70 and HMGB1, HSP70 antibody (Abcam, 1 : 1000) and HMGB1 antibody (Abeam, 1 : 1000) were applied to the membrane and maintained at 4°C overnight. Samples were washed with TBST, reacted with an HRP-conjugated anti-rabbit secondary antibody, and then analyzed.

As a result, RSVF-Exo increased CRT expression on the cell surface, and increased HSP70 and HMGB1 which are released from CT26.CL25 cells (FIGS. 6B-6C, FIG. 7D).

In addition, BMDM was stained with 1 mM CellTracker^{™} Green CMFDA dye (Thermo fisher Scientific). Cancer cells were treated with exosomes, seeded in 6-well plates, and then incubated at 37°C for 24 hours to induce immunogenic cell death (ICD). The exosome-treated cancer cells were stained with 1 mM pHrodo^{™} red, succinimidyl ester (Thermo fisher scientific). For phagocytosis assay, 2 × 10⁵ BMDM and 3.75 × 10⁵ cancer cells were co-cultured at 37°C for 2 hours. After washing with pH 10 PBS, phagocytosis (engulfment) of cancer cells by macrophages was visualized with a fluorescence microscope (Nikon Eclipse Ti, Nikon) and quantified using imaged.

As a result, RSVF-Exo-treated LLC and CT26.CL25 cancer cells had significantly high phagocytosis rates, as compared to the Con-Exo-treated control (FIG. 7E).

These results indicate that RSVF-Exo not only induces cancer cell death but also activates the host's immune system against cancer.

### Example 6. Tumor regression effect of RSVF-Exo

To investigate the antitumor effect of RSVF-Exo, the tumor suppression effect of RSVF-Exo *in vivo* was examined using two kinds of CT26.CL25 tumor-bearing mouse models.

In detail, 5 × 10⁵ CT26.CL25 cells were inoculated into 8-week-old male BALB/c mice, and on days 7 and 8, 100 µg of RSVF-Exo, Con-Exo, or PBS was intratumorally injected (i.t.) every day a total of twice.

To examine RSVF-Exo-induced CD8+ T cell immunity that increases efficacy of an immune checkpoint blockade therapy, a combination therapy of RSVF-Exo and anti-PD-1 blockade was performed on CT26.CL25 tumor-bearing BALB/c mice.

In detail, with regard to the combination therapy with anti-PD-1 antibody, CT26.CL25 tumor-bearing mice were injected with 100 µg of RSVF-Exo, Con-Exo, or PBS every day a total of three times on days 6 to 8 after tumor inoculation, and on days 11, 13, 15, and 17, 200 µg of anti-PD-1 antibody was injected.

As a result, when RSVF-Exo was treated, about 70% recovery effect on tumor size was observed (FIGS. 8A-8B), and there was no significant difference in the body weight (FIG. 9A).

It is known that RSVF which is a viral antigen interacts with toll-like receptor 4 (TLR4) and activates downstream signaling pathways, leading to activation of dendritic cells (DC) essential for T cell priming. Thus, it was investigated whether RSVF-Exo is able to induce tumor-specific immunity.

In detail, for interferon (IFN)-γ analysis, a CT26.CL25 tumor-bearing mouse-derived tumor-draining lymph node (TDLN) was prepared as a single cell suspension using a syringe plunger. After lysing red blood cells (RBC) in the single cell suspension using an RBC lysis buffer (Biolegend), 5 × 10⁵ TDLN cells were seeded in a 96-well plate. 5 µg/ml of gp70 and β-galactose were treated in each well, and reacted at 37°C for 24 hours. The amount of IFN-γ in the cell supernatant was measured using a mouse IFN-γ Quantikine ELISA kit (R & D Systems).

In addition, the single cell suspension of TDLN was pelleted by centrifugation. Single cells of TDLN were resuspended in PBS containing purified rat anti-mouse CD16/CD32 antibody (BD Biosciences, 1:100) for Fc blocking. Single cell suspension of TDLN was analyzed by flow cytometry using APC anti-mouse CD11c (Biolegend, 1:80), PE anti-mouse CD40 (Biolegend, 1:40), and PE anti-mouse CD86 (Biolegend, 1:40).

As a result, TDLN isolated from RSVF-Exo-treated mice showed an increased amount of IFN-γ secreted against tumor-specific peptides gp70 and β-galactose, as compared to the control group (FIG. 8C).

In addition, the expression levels of CD40 and CD86, which are dendritic cell maturation markers, in CD11c+ cells were increased in the RSVF-Exo treated group (FIG. 8D).

Meanwhile, in order to measure infiltration of CD8+ T cells in CT26.CL25 tumor-bearing BALB/c mouse-derived tumor tissue, the harvested tumor was dissociated with a tumor dissociation kit (Miltenyi Biotec, 130-096-730) and a dissociator (gentle MACS Octo Dissociator with Heaters, Miltenyi Biotec, 130-096-427) to prepare a single cell suspension. RBCs in the single cell suspension were lysed, and dead cells were removed using a dead cell removal kit. After treatment of the suspension with APC anti-CD3 (Biolegend, 1:40), PerCP anti-CD45.2 (Biolegend, 1:80), and FITC anti-CD8α (Biolegend, 1:50) antibodies, flow cytometry was performed to measure the amount of CD8+ T cells. Frozen tumor sections were washed with PBS and treated with CD8α antibody (BD Pharmingen, 1:200). After washing with PBS, the samples were stained with an Alexa Fluor 488-conjugated secondary antibody (1:400), followed by immunofluorescence analysis. The number of CD8+ T cells was counted using a confocal microscope.

As a result, the number of CD8+ T cells increased in the tumor tissues of the RSVF-Exo treated group (FIG. 8E).

These results indicate that RSVF-Exo is able to suppress tumor growth in *vivo* by activating the anti-tumor immune response.

### Example 7. Tumor site accumulation effect of RSVF-Exo

It was confirmed that RSVF-Exo is able to selectively fuse with cancer cells in a nucleolin-dependent manner, and thus it was examined whether RSVF-Exo intravenously injected has the tumor-targeting ability to accumulate only in the tumor site, not in an unintended organ.

In detail, RSVF-Exo was labeled with cyanine 5.5 NHS ester (Cy5.5-NHS) to measure biodistribution of exosomes. 300 µg of RSVF-Exo and Con-Exo were mixed with 3 µg of Cy5.5-NHS, and incubated at 4°C overnight, respectively. The labeled exosomes were centrifuged for 1 hour to remove unconjugated Cy5.5-NHS, and the exosome pellet was resuspended in PBS. Before injecting the labeled exosomes into mice, the Cy5.5-NHS conjugated exosomes were analyzed using a microplate reader to adjust the fluorescence intensity between samples.

The left flank of 8-week-old male BALB/c nude mice was inoculated with 5 × 10⁵ CT26.CL25 or 1 × 10⁷ LLC cells. When the average tumor volume reached 150 mm³, 300 µg of Cy5.5-labeled exosome or PBS was injected into tumor-bearing mice via the intravenous route. 5 minutes, 2 hours, 4 hours, 8 hours, 18 hours, and 24 hours after exosome injection, biodistribution of the exosomes was visualized through the IVIS spectrum (Caliper Life Sciences, IVIS^{®} Lumina Series III). Mice were sacrificed 24 hours after exosome injection, and ex *vivo* images were obtained by extracting lungs, livers, spleens and tumors.

As a result, visualized whole-body images showed that RSVF-Exo accumulated more at the tumor site than Con-Exo (FIGS. 10A and 10C). In addition, RSVF-Exo targeted the tumor site, as compared to Con-Exo, and there was no significant difference in the amount of Cy5.5-NHS signal between major organs such as lung, liver, and spleen (FIG. 10B).

These results were also confirmed in the LLC model (FIGS. 11A-11C).

### Example 8. Tumor regression effect of intravenous injection of RSVF-Exo

It was analyzed whether RSVF-Exo is able to suppress tumor growth *in vivo* through anti-tumor immunity when intravenously injected.

In detail, CT26.CL25 tumor-bearing BALB/c mice were intravenously injected with 100 µg of RSVF-Exo, Con-Exo, or PBS every day a total of three times on day 6 after inoculation of 1 × 10⁶ CT26.CL25 cells.

Spleen and TDLN extracted from CT26.CL25 tumor-bearing mice were prepared as single cell suspensions. RBCs in the single cell suspension were lysed using an RBC lysis buffer. After removing the RBC lysis buffer by centrifugation, the pellet was resuspended in PBS containing purified rat anti-mouse CD16/CD32 antibody (1:100). To evaluate CD8+ T cell activation in TDLN and spleen, single cell suspensions of TDLN and spleen were treated with APC anti-CD3 (1:40), PerCP anti-CD45.2 (1:80), and FITC anti-CD8α (1 :50) antibodies. In addition, PE anti-mouse/human CD44 antibody (Biolegend, 1:80) was added to TDLN samples, and PE anti-mouse CD154 antibody (Biolegend, 1:80) was added to spleen samples.

As a result, the RSVF-Exo-treated group suppressed tumor growth more than the control group, and there was no difference in the body weight between the groups (FIGS. 12A and 13A).

The dendritic cell maturation markers, CD40 and CD86 in TDLN were upregulated in the RSVF-Exo treated group (FIG. 12C).

As a result, RSVF-Exo treatment increased the frequency of CD8+ T cells in TDLN and the activation marker CD40 ligand (CD40L) on CD8+ T cells in the spleen (FIGS. 13B-13C).

In addition, the expression and frequency of CD44 on CD8+ T cells were evaluated to determine whether RSVF-Exo promotes CD8+ T cell memory. As a result, RSVF-Exo effectively increased the proportion of antigen-experienced CD44 expressing CD8+ T cells in TDLN (FIG. 12C). In addition, the amount of tumor-infiltrating CD8+ T cells was significantly increased by RSVF-Exo (FIG. 12C).

These results show that even when injected intravenously, RSVF-Exo is accumulated at the tumor site not only to suppress tumor growth *in vivo* through anti-tumor immunity, but also to promote the frequency and activation of CD8+ T cells and to improve the CD8+ T cell memory, thereby significantly increasing the amount of tumor-infiltrating CD8+ T cells.

### Example 9. Tumor regression effect of combination administration of RSVF-Exo

To examine RSVF-Exo-induced, CD8+ T cell immunity that increases efficacy of an immune checkpoint blockade therapy, a combination therapy of RSVF-Exo and anti-PD-1 blockade was performed on CT26.CL25 tumor-bearing BALB/c mice.

In detail, 1×10⁶ CT26.CL25 cells were inoculated into 8-week-old male Balb/c mice, and on days 7, 8, and 9, 100 ug of RSVF-Exo, Con-Exo, or PBS was injected every day a total of three times.

With regard to the combination therapy with anti-PD-1 antibody, the CT26.CL25 tumor-bearing mice were intratumorally injected with 100 µg of RSVF-Exo, Con-Exo, or PBS every day a total of three times on days 6 to 8 after tumor inoculation, and 200 µg of anti-PD-1 antibody was injected on days 11, 13, 15, and 17. Alternatively, the CT26.CL25 tumor-bearing mice were intravenously injected with 150 µg of RSVF-Exo, Con-Exo, or PBS every day a total of three times on days 6 to 8 after tumor inoculation, and on days 11, 13, 15, and 17, 200 µg of anti-PD-1 antibody was intraperitoneally injected (i.p.) a total of four times.

As a result, the anti-PD-1 antibody monotherapy group and the Con-Exo or PBS-administered control group showed no tumor suppression effect. In contrast, the combination therapy group of RSVF-Exo and anti-PD-1 antibody showed a synergistic effect on tumor suppression and improved the survival rate of mice (FIGS. 8F-8I, FIGS. 12E-12F).

These results indicate that systemic administration of RSVF-Exo via intravenous injection may suppress tumor growth without toxicity, unlike oncolytic virus, and may enhance the therapeutic effect of immune checkpoint blockade.

As shown in the results of Examples, RSVF-Exo kills cancer cells by specifically fusing to membranes of cancer cells, and suppresses tumor growth by accumulating in a tumor specific manner, and accordingly, it may be used for preventing or treating cancer.

Based on the above description, it will be understood by those skilled in the art that the present invention may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. An exosome expressing a respiratory syncytial virus F protein (RSVF).

2. The exosome of claim 1, wherein unfolded-proteins are accumulated inside the exosome due to inhibition of phagosome-lysosome fusion by treatment with a phagosome-lysosome fusion inhibitor.

3. The exosome of claim 2, wherein the inhibition of phagosome-lysosome fusion by the phagosome-lysosome fusion inhibitor is induced by any one selected from the group consisting of promotion of vacuolar type H+ ATPase, inhibition of proteasome function, inhibition of autophagosome formation, and deacetylase inhibition.

4. The exosome of claim 1, wherein the exosome specifically fuses to the membrane of cancer cells.

5. The exosome of claim 1, wherein the exosome kills cancer cells through immunogenic cell death.

6. The exosome of claim 1, wherein the exosome is accumulated in a tumor-specific manner to suppress tumor growth.

7. A pharmaceutical composition for preventing or treating cancer, the pharmaceutical composition comprising the exosome of any one of claims 1 to 6 as an active ingredient.

8. The pharmaceutical composition of claim 7, wherein the cancer is any one or more selected from the group consisting of esophageal cancer, stomach cancer, colon cancer, rectal cancer, oral cancer, pharyngeal cancer, larynx cancer, lung cancer, colon cancer, breast cancer, cervical cancer, endometrial cancer, ovarian cancer, prostate cancer, testicular cancer, bladder cancer, kidney cancer, liver cancer, pancreatic cancer, bone cancer, connective tissue cancer, skin cancer, brain cancer, thyroid cancer, leukemia, Hodgkin's disease, lymphoma, and multiple myeloma blood cancer.

9. A method of treating cancer, the method comprising the step of administering the pharmaceutical composition of claim 7 to an individual.

10. A vaccine composition for preventing cancer, the vaccine composition comprising the exosome of any one of claims 1 to 6 as an active ingredient.

11. A kit for preventing or treating cancer, the kit comprising a first composition including an exosome expressing a respiratory syncytial virus F protein; and a second composition including an anticancer agent as an active ingredient.

12. The kit of claim 11, wherein the administration route and frequency of the first composition and the second composition are independent of each other.

13. A method of preparing an exosome expressing a respiratory syncytial virus F protein, the method comprising the steps of:
i) transfecting cells with a plasmid including a polynucleotide encoding the respiratory syncytial virus F protein (RSVF);
ii) treating the cells of the step i) with a drug; and
iii) separating the exosome from the supernatant of the cells of the step ii).

14. The method of claim 13, wherein the drug is any one or more selected from the group consisting of a phagosome-lysosome fusion inhibitor, a proteasome function inhibitor, and an autophagosome function inhibitor.

15. The method of claim 14, wherein the phagosome-lysosome fusion inhibitor is any one or more selected from the group consisting of bafilomycin A1, chloroquine, hydroxychloroquine, quinacrine, Monensin, E64d, and pepstatin A.

16. The method of claim 14, wherein the proteasome function inhibitor is any one or more selected from bortezomib and carfilzomib.

17. The method of claim 14, wherein the autophagosome function inhibitor is any one or more selected from verteporfin and vinca alkaloids.
